# EUROPEAN PATENT APPLICATION

(11) **EP 3 138 553 A1**
(43) Date of publication of application: **08.03.2017**
(21) Application number: 16194441.8
(22) Date of filing: 11.04.2011
(51) Int. Cl.: A61K 9/06, A61K 9/107, A61K 9/20, A61K 31/343, A61K 31/496, A23K 20/174, A23K 20/158, A23K 20/195, A23K 50/40

(54) **ORAL VETERINARY PHARMACEUTICAL AND NUTRACEUTICAL COMPOSITIONS**

(30) Priority: 14.04.2010 GB 201006178
(62) Divisional of application: 11715599.4
(71) Applicant: Ayanda Group AS, 0667 Oslo (NO)
(72) Inventor: DRAGET, Kurt Ingar, 0667 Oslo (NO); HAUG, Ingvild Johanne, 0667 Oslo (NO); ENGELSEN, Steinar Johan, 0667 Oslo (NO); SETERNES, Tore, 0667 Oslo (NO)
(74) Representative: Robinson, Alexander Joseph

(57) **Abstract**

The invention provides an oral veterinary nutraceutical composition comprising a physiologically tolerable gelled oil-in-water emulsion further comprising at least one component selected from taste enhancers, odour enhancers, digestive enzymes and veterinary drugs. The invention further provides the use of a dietary supplement for the manufacture of a composition for use in a method of dietary supplementation of a non-human mammalian animal subject.

## Description

This invention relates to pharmaceutical and nutraceutical compositions for oral administration to non-human animals, especially mammals, more particularly domestic pets, especially carnivorous pets, e.g. dogs and cats.

Oral veterinary drugs are routinely presented in the same forms as oral drugs for humans, i.e. as tablets. Many animals however are highly adept at avoiding consuming tablets, even where camouflaged within their feed.

Domestic animals moreover frequently suffer from diseases associated with lipid and vitamin malnutrition. This can be caused by disease of the small intestine or exocrine pancreatic insufficiency (EPI) and may be treated by providing a diet enriched with highly digestible fats, nutritional supplementation with pancreatic extracts and vitamin supplementation. EPI, which is more common with dogs than cats, is frequently caused by pancreatic acinar atrophy in dogs and by pancreatitis in cats. Impaired fat uptake is also associated with impairment of uptake of fatsoluble vitamins.

We have now found that oral administration of drugs (pharmaceuticals) or nutritional supplements (nutraceuticals), such as vitamins, minerals, lipids, and digestive enzymes, may be facilitated by presenting the pharmaceutical or nutraceutical composition in the form of a physiologically tolerable, gelled, oil-in-water emulsion. The uptake of lipid nutrients from such compositions has moreover surprisingly been found to be higher with such gelled emulsions than with lipid-filled capsules. Due to their more food-like texture, and preferably enhanced with taste or odour attractants, consumption avoidance may be reduced or eliminated.

Thus viewed from one aspect the invention provides an oral veterinary pharmaceutical or nutraceutical composition comprising a physiologically tolerable gelled oil-in-water emulsion further comprising at least one component selected from taste enhancers, odour enhancers, digestive enzymes and veterinary drugs.

Viewed from a further aspect the invention provides an oral veterinary pharmaceutical composition comprising a physiologically tolerable gelled oil-in-water emulsion further comprising a veterinary drug for use in treatment of a non-human mammalian subject to combat a condition responsive to said veterinary drug.

Viewed from a still further aspect the invention provides a method of treatment of a non-human mammalian subject by oral administration to said subject of an effective amount of a veterinary drug, the improvement comprising administering said drug in a physiologically tolerable gelled oil-in-water emulsion.

Viewed from a further aspect the invention provides a method of dietary supplementation of a non-human mammalian animal subject which comprises orally administering to said subject at least one dietary supplement in a physiologically tolerable gelled oil-in-water emulsion.

The compositions of and used in the methods of the invention preferably contain an attractant, a material the taste or odour of which is attractive to the animal recipient. Typically this will be an extract from a prey animal, especially a proteinaceous and/or lipid material, e.g. a chicken, beef, pork, lamb, rabbit or fish flavouring, or a protein hydrolysate or oligopeptide, e.g. an animal or microbial protein hydrolysate. Where the animal recipient is feline, the attractant may conveniently be a fish oil, e.g. salmon oil.

Examples of nutrients that may be included in the compositions of the invention include lipids, (especially triglycerides and phospholipids, typically of plant or marine animal origin), vitamins, minerals, folic acid, and digestive enzymes, especially pancreatic digestive enzymes, e.g. lipases, proteases, amylases or a combination thereof.

Where the compositions are pharmaceuticals, i.e. where they contain a veterinary drug substance, this may be any veterinary drug substance that is suitable for oral administration. The drug substance may be presented in either or both of the water and oil phases of the gelled emulsion, e.g. in dissolved or dispersed form. The drug substance will typically be present in the gelled emulsion at 10 to 100% wt of the conventional oral daily dosage, especially 50 or 100% wt.

Examples of appropriate drug substances include antibiotics, antifungals, analgesics, antihelminthics, antidiabetics, oral contraceptives, hormones, antiinflammatory drugs, cardiovascular drugs, antihistamines, antidepressants and minerals, vitamins and essential fatty acids in triglyceride, monoacylglycerol, ethyl ester, phospholipid or free fatty acid form.

Particular examples of appropriate drug substances include: antibiotics e.g.penicillin V, cloxacillin, dicloxacillin, amoxicillin, ampicillin, cephalexin, cefaclor, cefdinir, doxycycline, doxycycline, enrofloxacin, oxytetracycline, azithromycin, erythromycin, clindamycin palmitate, trimethoprim, sulfamathoxazole (SMO), gentamicin, metronidazole, amoxycillin/clavulanic acid; antifungals e.g. polyene; antifungals e.g. amphotericin B, nystatin, pimaricin, imidazoles e.g. clotrimazole, miconazole, econazole, ketaconazole, itraconazole, fluoconazole, flucytocine and griseofulvin; analgesics e.g. morphine, butorphanol, hydromorphone, oxycodone; antihelminthics; clamoxyquine, dichlorophene, ivermectin, milbemycin oxime, ponazuril, mebendazole, flubendazole, fenbendazole, febantel, pyrantel tartrate, morantel, piperazine; antidiabetics e.g. glipizide; contraceptives e.g. megestrol acetate, mibolerone, proligestone, medroxyprogesterone acetate; hormones e.g. glucocorticoids, thyroid hormones, progesterones, sex hormones, melatonin; anti inflammatory drugs e.g. firocoxib, meloxicam, carprofen, ketoprofen, etodolac, aspirin; cardiovascular drugs e.g. digoxin,digitoxin, amrinone, milrinone, primobendan, enalapril, lisinopril, benazepril, hydralazine, amlodipine; antihistamines e.g. diphenhydramine, hydroxyzine, clorpheniramine, cyproheptadine, terfenadine, clemastine, trimeprazine; antidepressants like SSRI's; fluoxetine, paroxetine, certraline, citalopram; minerals and vitamins, e.g. zinc, calcium, retinyl palmitate, fat soluble vitamins (A, D, E, K) and water soluble vitamins (B-complex, C, folate etc) and essential fatty acids e.g. omega-3 fatty acids.

The gelled emulsion compositions of the invention will preferably be in dose unit form, with each dose unit having a weight of 50 to 5000 mg, especially 100 to 3000 mg, particularly 400 to 2000 mg, more particularly 600 to 1500 mg.

Although the compositions may be coated, e.g. as described in WO2007/085835, in order that the attractant should immediately be perceptible to the animal recipient, the dose units of the composition of the invention will preferably be uncoated, i.e. not within a capsule or shell-coating. Accordingly, to avoid water loss during storage, the dose units will conveniently be individually packaged, e.g. in foil wrappers or in the blisters of a blister pack.

Where a coating is used, this is preferably a gelatin coating. This can be applied before or after the emulsion has set.

Besides the components already mentioned, the compositions of the invention may optionally contain pH modifiers, sugars or other carbohydrates, viscosity modifiers, and colorants.

The oil phase of the oil-in-water emulsion may be any physiologically tolerable lipid, e.g. free fatty acids (or salts thereof), fatty acid esters such as mono-, di- and triacylglycerides and phospholipids, for example plant or animal oils, especially plant and marine animal oils. Particularly preferably an oil is used which is high in omega-3, omega-6 or omega-9 essential fatty acids, especially omega-3 essential fatty acids, more especially EPA and DHA. In this way the oil phase itself is a highly bioavailable source of nutrient lipids. It is especially preferred that the oil phase include free essential fatty acids (or physiologically tolerable salts thereof) and/or monoacylglycerides of essential fatty acids to facilitate essential fatty acid uptake from the gut.

Examples of omega-3 acids include α-linolenic acid (ALA), stearidonic acid (SDA), eicosatrienoic acid (ETE), eicosatetraenoic acid (ETA), eicosapentaenoic acid (EPA), docosapentaenoic acid (DPA), docosahexaenoic acid (DHA), tetracosapentaenoic acid and tetracosahexaenoic acid. Examples of omega-6 acids include linoleic acid, gamma-linolenic acid, eicosadienoic acid, dihomo-gamma-linolenic acid (DGLA), arachidonic acid (AA), docosadienoic acid, adrenic acid, docosapentaenoic acid, and calendic acid. Examples of omega-9 acids include oleic acid, eicosenoic acid, mead acid, erucic acid and nervonic acid.

The essential fatty acids may form part or the whole of the oil phase in the gelled emulsion, preferably at least 10% wt, more especially at least 50% wt, particularly at least 80% wt. of that phase. They may be used as single compounds or as compound mixtures, e.g. plant or marine oils.

Where a free fatty acid is used, this is preferably wholly or partially in salt form, and preferably constitutes 5 to 75% wt, especially 10 to 35% wt. of the essential fatty acid in the oil phase. Quite surprisingly, the soapy taste of the free fatty acid is masked by presentation in the gelled oil-in-water emulsion form. Where free fatty acids or salts thereof are used, it is particularly preferable to use these in conjunction with monoacylglycerides or diacylglycerides such that the molar ratio of free fatty acid to monoacylglyceride is about 2:1, e.g. 1.9:1 to 2.1:1, or free fatty acid to diacylglyceride is about 1:1, e.g. 0.9:1 to 1.1:1.

The oil phase of the oil-in-water emulsion may also contain solubilisers in order to increase the solubility of the drug substance in the oil phase. Suitable solubilisers would be known to a person skilled in the art and include Chremophor EL™, castor oil, Tween 80™, Solutol™ HS15, Lutrol™ and Olestra.

The aqueous phase of the gelled emulsion will contain water and a physiologically tolerable gelling agent, e.g. a hydrocolloid such as gelatin, alginate, carrageenan or a pectin. Such gelling agents and their gel-forming properties are well known. See for example Phillips GO and Williams PA (Eds.) Handbook of hydrocolloids, Woodhead Publishing, Cambridge (2000). When the compositions of the invention comprise enzymes such as one or more of lipases, proteases and amylases, the gelling agent is preferably pectin. The use of gelatin is especially preferred for use in the invention. Besides water and the gelling agent, the aqueous phase of the gelled emulsion may contain other water-soluble components, e.g. vitamins, minerals, pH modifiers, viscosity modifiers, antioxidants, colorants, flavours, water-soluble drug substances, etc. as desired.

Particularly preferably the gelled emulsions contain vitamins and optionally also minerals. Examples of such components include calcium, folic acid, iron, vitamin B12 and other B vitamins complexes, and vitamins A, D, E, K, and retinyl palmitate. Desirably these should be included at 10 to 100% of the recommended daily dose. If the composition contains fish oils, iron is less preferred due to its potentially oxidative effects.

The weight ratio of the lipid phase to the aqueous phase in the gelled emulsions is preferably 1:19 to 3:1, especially 35:65 to 1:1, particularly 2:3 to 1:1.

Emulsion formation may be effected by conventional techniques; however emulsification under a non-oxidising gas, e.g. nitrogen, is preferred. Likewise, the components of the emulsion are preferably degassed before emulsification and handling and packaging of the set emulsion is preferably performed under such a gas.

The gelled emulsions of and used according to the invention may be produced as described in WO 2007/085835 and WO 2007/085840 and PCT/GB2009/002404 and PCT/GB2009/002406 (copies of which are annexed to the description of this application as Annexes A and B) the contents of which are hereby incorporated by reference.

The gelled emulsions may if desired be more than biphasic. Thus a water-in-oil emulsion may be emulsified with an aqueous gelling agent phase to produce a water-in-oil-in-water double emulsion, or two oil-in-water emulsions with different oil phases may be combined and intimately mixed before gelling onset.

The invention will now be illustrated further with reference to the following nonlimiting Examples.

### Example 1

### Basic recipe for veterinary dosage form:

| Components (% bv wt.) | |
|---|---|
| Gelatin | 7.5 % |
| Gum arabicum | 3.5 % |
| Fish protein hydrolysate | 0-22 % |
| Chicken broth powder | 0-22 % |
| Salt | 0-2 % |
| Flavouring | 1 % |
| Colour | 0.5 % |
| Preservatives | 0-3 % |
| Oil | 5 to 35% |
| Water to | 100 % |

Oil (e.g. plant or marine animal oil) is emulsified with the aqueous phase containing gelatin, gum arabicum, proteins, salt and preservatives and the emulsion is poured in aliquots of 1.5 g into elongate moulds lined with a metal/plastics laminate blister tray and allowed to set. The blister tray is thermally sealed with a metal/plastics foil cover sheet.

### Example 2

### Canine and Feline Nutraceutical with essential fatty acids

| Components (% bv wt.) | |
|---|---|
| Gelatin | 7.5 % |
| Gum arabicum | 3.5 % |
| Fish protein hydrolysate | 0-22% |
| Chicken broth powder | 0-22% |
| Salt | 0-2% |
| Flavouring | 1% |
| Colour | 0.5 % |
| Preservatives | 0-3% |
| Triglycerides* | 0-40 % |
| Ethyl esters of fatty acids* | 0-40% |
| Free fatty acids* | 0-40% |
| Monoacylglycerides* | 0-40% |
| Phospholipids* | 0-40% |
| Water to | 100% |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 3

### Canine and Feline Nutraceutical with essential fatty acids and vitamins

| Components (% by wt.) | |
|---|---|
| Gelatin | 7.5 % |
| Gum arabicum | 3.5 % |
| Fish protein hydrolysate | 0-22% |
| Chicken broth powder | 0-22% |
| Salt | 0-2% |
| Flavouring | 1 % |
| Colour | 0.5 % |
| Preservatives | 0-3% |
| Triglycerides* | 0-40% |
| Ethyl esters of fatty acids* | 0-40% |
| Free fatty acids* | 0-40 % |
| Monoacylglycerides* | 0-40% |
| Phospholipids* | 0-40% |
| Vitamins | 0.01-1% |
| Water to | 100 % |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified together with the lipid-soluble vitamins into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 4

### Canine and Feline antibiotics

| Components (% by wt.) | |
|---|---|
| Gelatin | 7.5 % |
| Gum arabicum | 3.5 % |
| Fish protein hydrolysate | 0 - 22 % |
| Chicken broth powder | 0 - 22 % |
| Salt | 0 - 2 % |
| Flavouring | 1 % |
| Colour | 0.5 % |
| Preservatives | 0 - 3 % |
| Oil | 5 to 35 % |
| Ciprofloxacin | 180 mg/tablet* |
| Water to | 100 % |

| | |
|---|---|
| * Suitable for a 15kg animal - to be adjusted for smaller/heavier animals. | |

Oil (e.g. plant or marine animal oils), together with ciprofloxacin, is emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 5

### Canine and Feline antifungals

| Components (% by wt.) | |
|---|---|
| Gelatin | 7.5 % |
| Gum arabicum | 3.5 % |
| Fish protein hydrolysate | 0 - 22 % |
| Chicken broth powder | 0-22% |
| Salt | 0-2% |
| Flavouring | 1% |
| Colour | 0.5 % |
| Preservatives | 0-3% |
| Oil | 5 to 35% |
| Griseofulvin | 375 mg* |
| Water to | 100 % |

| | |
|---|---|
| * Suitable for a 15kg animal - to be adjusted for smaller/heavier animals. | |

Oil (e.g. plant or marine animal oils), together with griseofulvin is emulsified into the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 6

### Canine and Feline Nutraceutical with digestive enzyme and nutrients

| Components (% by wt.) | |
|---|---|
| Gelatin | 7.5 % |
| Gum arabicum | 3.5 % |
| Fish protein hydrolysate | 0-22% |
| Chicken broth powder | 0-22% |
| Salt | 0-2% |
| Flavouring | 1% |
| Colour | 0.5 % |
| Preservatives | 0-3% |
| Triglycerides* | 0-40% |
| Ethyl esters of fatty acids* | 0-40% |
| Free fatty acids* | 0-40% |
| Monoacylglycerides* | 0-40% |
| Phospholipids* | 0-40% |
| Vitamins | 0.01-1% |
| Pancreatic Lipase | 10 000 U |
| Water to | 100% |

| | |
|---|---|
| * Total not to exceed 40% | |

Free fatty acids (e.g. omega-3 acids, in particular a mixture of DHA and EPA), ethyl esters, triacylglycerides, phospholipids, and/or monoacylglycerides of poly and mono unsaturated fatty acids are emulsified together with the lipid-soluble vitamins into the aqueous phase with pancreatic lipase and the emulsion is poured in aliquots of 1.5 g into elongate moulds lined with a metal/plastics laminate blister tray and allowed to set. The blister tray is thermally sealed with a metal/plastics foil cover sheet.

Preferred embodiments of the inventions are as follows:
1. An oral veterinary pharmaceutical or nutraceutical composition comprising a physiologically tolerable gelled oil-in-water emulsion further comprising at least one component selected from taste enhancers, odour enhancers, digestive enzymes and veterinary drugs.
2. A composition as described in embodiment 1 containing a digestive enzyme,
3. A composition as described in either of embodiments 1 and 2 containing a protein hydrolysate.
4. A composition as described in any one of embodiments 1 to 3 containing a chicken flavouring.
5. A composition as described in any one of embodiments 1 to 3 containing a beef flavouring.
6. A composition as described in any one of embodiments 1 to 3 containing a lamb flavouring.
7. A composition as described in any one of embodiments 1 to 3 containing a rabbit flavouring.
8. A composition as described in any one of embodiments 1 to 3 containing a salmon flavouring.
9. A composition as described in any one of embodiments 1 to 3 containing a pork flavouring.
10. An oral veterinary pharmaceutical composition comprising a physiologically tolerable gelled oil-in-water emulsion further comprising a veterinary drug for use in treatment of a non-human mammalian subject to combat a condition responsive to said veterinary drug.
11. A composition as described in any of the preceding emdbodiments wherein the oil phase of said emulsion comprises an omega-3 fatty acid or ester or salt thereof.
12. A method of treatment of a non-human mammalian subject by oral administration to said subject of an effective amount of a veterinary drug, the improvement comprising administering said drug in a physiologically tolerable gelled oil-in-water emulsion.
13. A method as described in embodiment 12 being a method of treatment of a disease of the small intestine or exocrine pancreatic insufficiency.
14. A method of dietary supplementation of a non-human mammalian animal subject which comprises orally administering to said subject at least one dietary supplement in a physiologically tolerable gelled oil-in-water emulsion.

## Claims

1. An oral veterinary nutraceutical composition comprising a physiologically tolerable gelled oil-in-water emulsion further comprising at least one component selected from taste enhancers, odour enhancers, digestive enzymes and veterinary drugs.

2. A composition as claimed in claim 1 comprising an attractant which is a chicken, beef, pork, lamb, rabbit or fish flavouring.

3. A composition as claimed in claim 1 or claim 2 comprising a dietary supplement selected from folic acid, vitamins, minerals, lipid nutrients, or digestive enzymes.

4. A composition as claimed in any of claims 1 to 3 wherein the aqueous phase of the gelled oil-in-water emulsion comprises a physiologically tolerable gelling agent selected from gelatin, alginate, carrageenan and a pectin.

5. A composition as claimed in any of claims 1 to 4 containing a protein hydrolysate.

6. A composition as claimed in any one of claims 1 to 5 containing a salmon flavouring.

7. A composition as claimed in any of claims 1-6 wherein the oil phase of said emulsion comprises an omega-3 fatty acid or ester or salt thereof.

8. Use of a dietary supplement for the manufacture of a composition for use in a method of dietary supplementation of a non-human mammalian animal subject, wherein said composition comprises at least one dietary supplement in a composition as claimed in any one of claims 1 to 7 and wherein said method comprises orally administering said composition to said subject.

9. A dietary supplement for use in a method of dietary supplementation of a non-human mammalian animal subject, wherein said method comprises orally administering to said subject at least one dietary supplement in a composition as claimed in any one of claims 1 to 7.
